# EUROPEAN PATENT APPLICATION

(11) **EP 1 621 080 A1**
(43) Date of publication of application: **01.02.2006**
(21) Application number: 05254392.3
(22) Date of filing: 13.07.2005
(51) Int. Cl.: A23G 3/00

(54) **Edible film compositions**

(30) Priority: 30.07.2004 US 710744
(71) Applicant: WM. WRIGLEY JR. COMPANY, Chicago, Illinois 60611 (US)
(72) Inventor: Barkalow, David, Dearfield, Illinois 60015 (US); Zyck, Daniel, North Riverside, Illinois 60546 (US); Soto, Miguel, Chicago, Illinois 60633 (US)
(74) Representative: Hayes, Adrian Chetwynd

(57) **Abstract**

The present invention provides edible film formulations using low viscosity hydrolyzed vegetable gum as a film forming component.

## Description

### Background of Invention

Orally consumable films have gained acceptance in the consumer marketplace, delivering benefits such as breath freshening, oral health care, pharmaceutical delivery as well as flavour enjoyment. Such films may be prepared using a variety of materials as the primary film forming ingredients. While each of these materials has its advantages, each also has disadvantages, such as cost, availability, slow dissolution, gummy mouthfeel, lack of regulatory approval, stability, off-taste and difficulties in manufacturing. The present invention provides an improved oral film, which minimizes the disadvantages of prior film compositions.

Edible film products are known in the art. These products are designed to adhere to and rapidly dissolve in the mouth of the consumer. Edible films can provide flavour and/or oral care benefits, e.g., breath freshening to the consumer. Such films typically include a film former and flavour or other ingredients. See, for example, U.S. Patent No. 5,948,430 and U.S. Application Publication No. US2001/0022964 A1. Edible film products are typically provided to the consumer in strip form. The strips are usually sized so that they can be placed on the tongue of a consumer. In this regard, the edible film strips typically have a size of a postage stamp or slightly larger, although they may come in many different shapes and sizes. These strips preferably have a supple texture and are non-self adhering.

One type of edible film product is distributed by the Wm. Wrigley Jr. Company under the name of Eclipse Flash@ Strips. This edible film product is packaged in a plastic container that includes a top that can open along a hinge. A stack of strips is located in an interior of the package, one strip on top of another. The package is designed so that the consumer can open the container and remove one strip from the stack with his or her finger.

### SUMMARY OF INVENTION

It has now been discovered that high quality, low cost oral films can be economically compounded using low viscosity hydrolyzed vegetable gum as a film forming component. Films made according to the present invention dissolve quickly with reduced gumminess and off-flavours.

Additional features and advantages of the present invention will be described herein, and apparent from the detailed description of the invention.

### DETAILED DESCRIPTION

Generally, the present invention provides edible film formulations for oral mucoadhesion, and methods of using and making same. More specifically, the present invention provides edible film formulations containing low viscosity hydrolyzed vegetable gum as a film forming agent.

Edible films of the present invention employ an effective amount of a low viscosity hydrolyzed vegetable gum. Vegetable gums are polymeric carbohydrates derived from plant materials and are commonly used as additives in a variety of food products. Although less commonly used, these gums may be hydrolyzed by acids or enzymes to produce gums having lower molecular weight. Examples of such hydrolyzed vegetable gums which may be useful in the present invention include hydrolyzed guar gum, hydrolyzed locust bean gum, hydrolyzed larch gum, hydrolyzed carrageenan, hydrolyzed gum arabic, hydrolyzed sodium alginate and hydrolyzed gum tragacanth. In an embodiment, hydrolyzed vegetable gums used in the present invention are galactomannans. In an embodiment, hydrolyzed galactomannan is enzymatically hydrolyzed guar gum which is produced by Taiyo Kagaku Co., Ltd. and marketed in the U.S. by Novartis of Minneapolis, Minn. Under the trade name Benefiber®. The use of low viscosity hydrolyzed vegetable gums provide an edible film with characteristics such that the films dissolve quickly with reduced gumminess and no off-flavours.

Hydrolyzed guar gum is in a family of vegetable gums called galactomannans. These materials are made up of mannose and galactose units. The main chain consists of (1->4)-linked beta-D-mannose residues and the side chains of (1->6)-linked alpha-D-galactose. Locust bean gum is another galactomannan. Galactomannans themselves are part of a larger group of natural gums. PCT Publication No. WO 93/15116 discloses a process for hydrolyzing guar gum, locust bean gum and karaya gum, which are characterized as "natural carbohydrate" gums. The other hydrolyzates disclosed in the PCT Publication may be useful in a manner similar to guar gum hydrolyzate.

A preferred low viscosity hydrolyzed vegetable gum used in the present invention is hydrolyzed guar gum. This is a flavourless, colorless, fine white powder, and is a soluble dietary fiber and is unnoticeable when added to food products. Viscosity comparisons were made between a hydrolyzed guar gum, Benefiber® and sodium alginate. Test method was using a Brookfield DVII Viscometer, using Spindle #2, at 25 degrees centigrade and sample dilutions at 15% in water. The temperature was held at 25 degrees centigrade using a jacketed beaker connected to a waterbath. The rpm adjusted per sample, to target a % accuracy of the instrument close to 50%. Using this method, the viscosity of hydrolyzed guar gum at 100 rpm was 82.4 cps. The viscosity of the sodium alginate at 2.5 rpm was 32900 cps. Not wishing to be bound by theory, it is believed that the low viscosity of the hydrolyzed guar gum, when used in an edible film formulation, decreases the gummy mouthfeel experienced with edible film formulations presently on the market. Provided the method described above, and for purposes of the present invention, a low viscosity may be defined as up to 10,000 cps. In an embodiment of the present invention, an edible film comprises a low viscosity hydrolyzed vegetable gum having viscosity up to 10,000 cps. More preferably, an edible film comprises a low viscosity hydrolyzed vegetable gum having viscosity up to 5000 cps. Most preferably, an edible film comprises a low viscosity hydrolyzed vegetable gum having viscosity up to 500 cps.

These hydrolyzed vegetable gums are film forming agent, which may be used as the only film forming agent, or in combination with other film forming agents, at levels ranging from 2% to 60% by weight (dry basis) in the films. In an embodiment, these hydrolyzed vegetable gums may be employed at levels ranging from 15% to 50% by weight. In yet another embodiment, the hydrolyzed vegetable gums will be employed at levels ranging from 20% to 45% by weight.

Typically, other ingredients will be incorporated into the edible films of the present inventions. Other ingredients may include additional film forming agents, fillers, plasticizers, flavours, emulsifiers, colors, sweeteners, high-intensity sweeteners, acids etc. Further, the edible films may include a variety of other suitable ingredients, such as softeners, heating agents, cooling agents, surfactants, thickening agents, binding agents, active agents, fragrances, other like ingredients or combinations thereof.

Additional film forming agents which may be used in the present invention include sodium alginate, carrageenan, Pullulan, modified starch (e.g. hydroxypropyl starch), hydroxypropyl methylcellulose (HPMC), pectin, hydrolyzed alginates, polysaccharides, maltodextrin, starch, gum arabic, guar gum, larch gum, locust bean gum, xanthan gum, hydrocolloids, and combinations thereof. Such film formers also modify the texture of the edible film. The selection of film formers may make the edible film more or less brittle, and also contribute to modifying the gummy properties of edible films. The total film forming agent may be present at a level of 2% to 60%, more preferably in amounts from 20% to 45% of the total film forming composition.

A hydrocolloid, mentioned above, may be derived from, for example, natural seaweeds, natural seed gum, natural plant exudates, natural fiber extracts, biosynthetic gums, gelatins, biosynthetic process starch or cellulosic materials, alginates, sodium alginate, calcium alginate, carrageenans, guar gum, locust gum, larch gum, tara gum, gum arabic, ghatti gum, agar gum, xanthan gum, pectin, other like hydrocolloid source materials or combinations thereof. The hydrocolloid, in addition to being a film former, can provide thickness and decrease brittleness of the edible films, as mentioned above. The hydrocolloid can include any suitable type, amount and number of hydrocolloids.

Any suitable food-grade bulk filler can also be added to the edible film. This can reduce any slimy texture as well as provide structure to the film, thereby making it more palatable. In addition, fillers control gumminess and dissolution rate and they help keep the films separated from each other. In an embodiment, the filler can constitute 2% to 30% by dry weight of the film, preferably 5% to 15% by dry weight. The filler can include, for example, microcrystalline cellulose; cellulose polymers, such as wood; magnesium carbonate; calcium carbonate; ground limestone; silicates; clay; talc; titanium dioxide; monocalcium phosphate; di-calcium phosphate; tri-calcium phosphate; other like bulk fillers or combinations thereof.

If reduced levels of film forming agents are utilized, softeners may be used to reduce the brittleness of the resulting films. The softeners, which are also known as plasticizers or plasticizing agents, generally constitute up to 20% by dry weight of the film, preferably 2% to 10% by dry weight. The softeners can include plasticizers containing, for example, sorbitol and mixtures of sugar alcohols, xylitol, glycerin, polyethylene glycol, propylene glycol, hydrogenated starch hydrolysates, corn syrups, glycerin, triacetin, glycerol oleate, sucrose fatty acid ester, Neobee oil, medium chain triglycerides other like material or combinations thereof.

Coloring agents which may be used in the present invention may include, for example, natural food colors and dyes suitable for food, drug and cosmetic applications. The colors, typically knows as FD&C dyes and lakes, may be present in amounts from 0.01% to 1.5% by weight of the edible film formulation.

A variety of flavouring agents may also be added to the edible films. Any suitable amount and type of artificial and/or natural flavouring agents can be used in any sensorially acceptable fashion. For example, the flavour can constitute 0.1% to 20% by dry weight of the film, preferably 5% to 15%. The flavouring agents can include, for example, essential oils, synthetic flavours or mixtures, including but not limited to, oils delivered from plants and fruits such as citrus oils, fruit essences, peppermint oil, spearmint oil, other mint oils, clove oils, oil of wintergreen, anise and the like, flavour oils with germ killing properties such as menthol, eucalyptol, thymol, like flavouring agents or combinations thereof.

The flavour can be enhanced and evenly distributed throughout the product by emulsification. Any suitable amount and type of natural and/or synthetic food-grade emulsifier can be used. For example, the emulsifier can include lecithin, food-grade non-ionic emulsifiers, such as fatty acids (C₁₀-C₁₈), mono-glycerides, diacyl-glycerides, ox bile extract, polyglycerol esters, polyethylene sorbitan esters, propylene glycol, sorbitan monopalmitate, sorbitan monosterate, sorbitan tristerate, enzyme modified lecithin, hyroxylated lecithins, other like emulsifiers or combinations thereof. Emulsifiers may be used in amount ranging from 0.1% to 3%. The flavours can be emulsified by any suitable emulsification process, such as mechanical processing, vigorous stirring, intense pressure fluctuations that occur in turbulent flow such as homogenization, sonication, colloid milling and the like.

The bulk sweeteners, which may be added to the film composition, include both sugar and sugarless components. Sugar sweeteners generally include saccharide components commonly known in the art, including but not limited to, sucrose, dextrose, maltose, dextrin, dried invert sugar, fructose, levulose, galactose, corn syrup solids, and the like, along or in combination. Sugarless sweeteners include, but are not limited to, sugar alcohols such as sorbitol, mannitol, xylitol, hydrogenated starch hydrolysates, maltitol, and the like, alone or in combination.

High-intensity artificial sweeteners can also be used, alone or in combination, with the above. High-intensity sweeteners include, but are not limited to, sucralose, aspartame, NAPM derivatives such as neotame, salts of acesulfame, altitame, saccharin and its salts, cyclamic acid and its salts, glycyrrhizinate, neohesperidine, dihydrochalcones, thaumatin, monellin, and the like, alone or in combination. In order to provide longer lasting sweetness and flavour perception, it may be desirable to encapsulate or otherwise control the release of at least a portion of the artificial sweetener.

Flavors may also be enhanced by the use of heating and cooling agents in the edible film formulations. Cooling agents enhance the flavour and perceived breath freshening of the product. Cooling agents include N-ethyl-p-menthane-3-carboxamide (WS-3), N,2,3-trimethyl-2-isopropyl-butanamide (WS-23), menthyl glutarate, menthyl succinate, menthol PG carbonate, menthol EG carbonate, menthyl lactate, menthone glyceryl ketal, menthol glyceryl ether, 3,3,5-trimethylcyclohexanol (Homomenthol), isopulegol and combinations thereof. Cooling agents may be used in amounts of 0.01% to 2% by weight of the edible film formulation.

The present invention may also optionally contain a heating agent. The heating agent, in small amounts, potentiates the effect of the cooling agent. The heating agent may also substitute for the cooling agent in the present invention if the experience of heat, tingling or itching is desired. The heating agents of the present invention are capsicum oleoresin, capsaicin, piperine, gingerol, shoagol, cinnamic aldehyde, ginger oleoresin, cinnamon oleoresin, cassia oleoresin, black pepper oleoresin, pepper oleoresin and combinations thereof. In the present invention, generally the heating agent may be used in amounts of 0.01% to 3% by weight of the film formulation.

A variety of other suitable ingredients can be added to the edible film of the present invention. For example, any suitable medicament for oral cleansing, breath freshening or the like can be added to the film formulation. The medicaments can include, for example, a pH control agent, such as urea and buffers; inorganic components for tartar or caries control, such as phosphates and fluorides; a breath freshening agent such as zinc gluconate; an anti-plaque anti-gingivitis agent, such as cholorhexidene, CPC, and triclosan; a saliva stimulating agent including, for example, food acids such as citric, lactic, maleic, succinic, ascorbic, adipic, fumaric and tartaric acids; a pharmaceutical agent; a nutraceutical agent; a vitamin; a mineral; other like medicaments or combinations thereof. The highly dissolvable edible film can act as a medium through which a pharmaceutically active oral agent can be administered via a mucous membrane of the oral cavity.

The present invention provides methods of producing the edible film formulations. In general, the edible film formulations are prepared by forming a base solution that includes ingredients such as maltodextrins, hydrocolloids and fillers, and processing the base solution to form an edible film. Typically, the base solution is prepared by adding an initial mixture of dry ingredients to water that is stirred.

In an embodiment, the solution is stirred continuously and heated at a temperature ranging from 40°C to 60°C. The solution then can be dried in any suitable manner, thereby forming the edible film.

It should be appreciated that any suitable type, number and arrangement of process procedures or steps (i.e. mixing, heating, drying, cooling, addition of ingredients), process parameters (i.e. temperature, pressure, pH, process times) or the like can be utilized.

By way of example and not limitation, examples of embodiments of edible films are provided herein.

An edible film formulation using hydrolyzed guar gum was made and compared to a control which did not contain hydrolyzed guar gum as a film former. The protocol by which the films were produced are following Table 1.

**Table 1. Film formulations (dry weight)**

| Ingredient | Control | Example A |
|---|---|---|
| Water | 10.00 | 10.00 |
| Sodium Alginate | 26.495 | 16.434 |
| Maltodextrin | 23.325 | 16.441 |
| Peppermint | 10.158 | 8.895 |
| Carrageenan | 8.548 | 0 |
| Microcrystalline | 7.040 | 7.262 |
| Cellulose | | |
| Glycerine 99% | 6.384 | 6.583 |
| Menthol | 3.565 | 3.126 |
| Sucralose | 3.095 | 2.714 |
| Hydroxylated Lecithin | 1.082 | 0.948 |
| Color | 0.308 | 0.270 |
| Benefiber®(hydrolyzed guar gum) | 0 | 27.327 |
| | | |
| Total | 100 | 100 |

Preparation of films in Table 1: Step 1 - Dry blend sodium alginate, maltodextrin, carrageenan and microcrystalline cellulose (and/or other hydrocolloids per formula in Table 1). Step 2 - Blend peppermint, menthol and hydroxylated lecithin. Step 3 - Add color to room temperature, deionized water. Step 4 - Add dry ingredients from step 1 to color water from step 3 (add slowly with stirring). Step 5 - Stir and warm to about 50°C until hydrocolloids dissolved (no lumps). Step 6 - Add glycerine and sucralose. Mix until dissolved. Step 7 - Add flavour blend from step 2 and stir until appearance is uniform. Step 8 - Warm glass plate and draw down bar in 55°C oven. Step 9 - Apply film solution to glass plate with draw down bar 0.279 - 0.381mm (0.011 - 0.015 inch; target weight = 0.05g per individual film serving). Step 10 - Dry in oven at 55°C (10-15 minutes). Do not over dry, or film will become brittle. Step 11 - Cut in rectangle shaped individual servings using grid guide. Step 12 - Pack in film container and place in zip-lock bag.

A descriptive panel was conducted to evaluate the hydrolyzed guar gum formula, Example A, against the Control. Example A showed no difference in cooling levels compared to the Control. However, Example A was quicker to dissolve, not as gummy and had a deeper flavour note as compared to the Control.

The hydrolyzed guar gum formulation was similar to control as far as curling, but was slightly more brittle. The hydrolyzed guar gum sample, Example A, was a promising alternative backbone formulation for fast dissolving films.

Additional film formulations, which include hydrolyzed vegetable gums, are presented in Table 2 and Table 3.

**Table 2. Examples of Film Formulations with Low Viscosity Hydrolyzed Vegetable Gum.**

| Ingredient | Example B | Example C | Example D | Example E |
|---|---|---|---|---|
| Water | 10.000 | 10.000 | 10.000 | 10.000 |
| Maltodextrin | 15.712 | 17.436 | 17.681 | 17.430 |
| Sodium Alginate | 14.885 | 8.469 | 12.797 | 1.494 |
| Microcrystalline Cellulose | 6.940 | 6.968 | 7.067 | 6.964 |
| Carrageenan | 1.819 | 2.591 | 3.940 | 5.976 |
| Hydrolyzed Guar | 29.108 | 32.913 | 26.588 | 36.519 |
| Gum | | | | |
| Glycerin | 6.291 | 6.317 | 6.405 | 6.315 |
| Lecithin | 0.906 | 0.910 | 0.923 | 0.910 |
| Color | 0.258 | 0.259 | 0.263 | 0.259 |
| Menthol | 2.987 | 2.998 | 3.041 | 2.998 |
| Flavor | 8.501 | 8.535 | 8.655 | 8.532 |
| Sucralose | 2.593 | 2.604 | 2.640 | 2.603 |
| | | | | |
| Total | 100 | 100 | 100 | 100 |

**Table 3. Examples of Film Formulations with Low Viscosity Hydrolyzed Vegetable Gum**

| Ingredient | Example F | Example G | Example H | Example I |
|---|---|---|---|---|
| Water | 10.000 | 10.000 | 10.000 | 10.000 |
| Maltodextrin | 15.597 | 15.919 | 16.048 | 16.048 |
| Sodium Alginate | 3.284 | 11.730 | 15.134 | 19.528 |
| Microcrystalline Cellulose | 6.889 | 7.033 | 7.088 | 7.088 |
| Carrageenan | 0.000 | 7.038 | 0.000 | 8.446 |
| Hydrolyzed Guar Gum | 42.851 | 26.460 | 29.731 | 16.891 |
| Glycerin | 6.245 | 6.374 | 6.426 | 6.426 |
| Lecithin | 0.900 | 0.918 | 0.926 | 0.926 |
| Color | 0.256 | 0.261 | 0.264 | 0.264 |
| Menthol | 2.965 | 3.026 | 3.051 | 3.051 |
| Flavor | 8.439 | 8.613 | 8.683 | 8.683 |
| Sucralose | 2.574 | 2.628 | 2.649 | 2.649 |
| | | | | |
| Total | 100 | 100 | 100 | 100 |

The advantages of the preferred embodiments of the present invention provide an economically priced, quickly dissolving edible film with improved mouthfeel, reduced gumminess and no off-flavors.

It should be understood that various changes and modifications to the presently preferred embodiments described herein will be apparent to those skilled in the art. Such changes and modificiations can be made without departing from the spirit and scope of the present invention and without diminishing its intended advantages. It is therefore intended that such changes and modifications be covered by the appended claims.

## Claims

1. An edible film composition comprising:
a) at least one film forming agent;
wherein said film forming agent comprises a low viscosity hydrolyzed vegetable gum.

2. The composition of claim 1, wherein said low viscosity hydrolyzed vegetable gum is selected from the group consisting of hydrolyzed guar gum, hydrolyzed locust bean gum, hydrolyzed larch gum, hydrolyzed carrageenan, hydrolyzed gum arabic, hydrolyzed gum tragacanth and combinations thereof.

3. The composition of claim 2, wherein said low viscosity hydrolyzed vegetable gum comprises hydrolyzed guar gum.

4. The composition of any one of claims 1 to 3, wherein said low viscosity hydrolyzed vegetable gum is present in an amount of 2% to 60% by weight of said edible film.

5. The composition of any one of claims 1 to 3, wherein said low viscosity hydrolyzed vegetable gum is present in an amount of 15% to 50% by weight of said edible film.

6. The composition of any one of claims 1 to 3, wherein said low viscosity hydrolyzed vegetable gum is present in an amount of 20% to 45% by weight of said edible film.

7. The composition of any one of the preceding claims, wherein said edible film further comprises a sweetener.

8. The composition of claim 7, wherein said sweetener is selected from the group consisting of sucrose, dextrose, maltose, dextrin, dried invert sugar, fructose, levulose, galactose, corn syrup solids, sugar alcohols such as sorbitol, mannitol, xylitol, hydrogenated starch hydrolysates, maltitol and combinations thereof.

9. The composition of any one of the preceding claims, wherein said edible film further comprises a high-intensity sweetener.

10. The composition of claim 9, wherein said high-intensity sweetener is selected from the group consisting of sucralose, aspartame, NAPM derivatives, salts of acesulfame, altitame, succharin and its salts, cyclamic acid and its salts, glycyrrhizinate, neohesperidine, dihydrochalcones, thaumatin, monellin and combinations thereof.

11. The composition of any one of the preceding claims, wherein said edible film further comprises at least one additional film forming agent.

12. The composition of claim 11, wherein said additional film forming agent is selected from the group consisting of sodium alginate, carrageenan, Pullulan, modified starch, hydroxypropyl methylcellulose, pectin, hydrolyzed alginates, polysaccharides, maltodextrin, starch, gum arabic, guar gum, larch gum, locust bean gum, xanthan gum, hydrocolloids and combinations thereof.

13. The composition of any one of the preceding claims, wherein said edible film further comprises a filler.

14. The composition of claim 13, wherein said filler is selected from the group consisting of microcrystalline cellulose, cellulose polymers, magnesium carbonate, calcium carbonate, ground limestone, silicates, magnesium silicate, aluminium silicate, clay, talc, titanium dioxide, monocalcium phosphate, di-calcium phosphate, tricalcium phosphate and combinations thereof.

15. The composition of claim 13 or 14, wherein said filler is present in the amount of 2% to 30% by weight of said edible film.

16. The composition of claim 13 or 14, wherein said filler is present in the amount of 5% to 15% by weight of said edible film.

17. The composition of any one of the preceding claims, wherein said edible film further comprises a softener.

18. The composition of claim 17, wherein said softener is selected from the group consisting of sorbitol, xylitol, glycerin, polyethylene glycol, propylene glycol, hydrogenate starch hydrolysates, corn syrups, glycerin, triacetin, glycerol oleate, sucrose fatty acid ester, Neobee oil and combinations thereof.

19. The composition of claim 17 or 18, wherein said softener is present in the amount of up to 20% by weight of said edible film.

20. The composition of claim 17 or 18, wherein said softener is present in the amount of 2% to 10% by weight of said edible film.

21. The composition of any one of the preceding claims, wherein said edible film further comprises a flavour.

22. The composition of any one of the preceding claims, wherein said edible film further comprises an emulsifier.

23. The composition of claim 22, wherein said emulsifier is selected from the group consisting of lecithin, fatty acids, mono-glycerides, diacyl-glycerides, polyglycerol esters, polyethylene sorbitan esters, propylene glycol, sorbitan monopalmitate, sorbitan monsterate, sorbitan tristerate, enzyme modified lecithin, hydroxylated lecithins, and combinations thereof.

24. The composition of claim 22 or 23, wherein said emulsifier is present from 0.1% to 3% by weight of said edible film.

25. The composition of any one of the preceding claims, wherein said edible film further comprises a cooling agent.

26. The composition of claim 25, wherein said cooling agent is selected from the group consisting of N-ethyl-p-menthane-3-carboxamide (WS-3), N,2,3-trimethyl-2-isopropyl-butanamide (WS-23), menthyl glutarate, menthyl succinate, menthol PG carbonate, menthol EG carbonate, menthyl lactate, menthone glyceryl ketal, menthone glyceryl ether, 3,3,5-trimethylcyclohexanol (Homomenthol), isopulegol and combinations thereof.

27. The composition of any one of the preceding claims, wherein said edible film further comprises a heating agent.

28. The composition of claim 27, wherein said heating agent is selected from the group consisting of capsicum oleoresin, capsaicin, piperine, gingerol, shoagol, cinnamic aldehyde, ginger oleoresin, cinnamon oleoresin, cassia oleoresin, black pepper oleoresin, pepper oleoresin and combinations thereof.

29. The composition of any one of the preceding claims, wherein said edible film further comprises an active agent.

30. The composition of claim 29, wherein said active agent is selected from the group consisting of a breath freshening agents, oral cleansing agents, tartar control agents, caries control agents, anti-plaque agents, saliva stimulating agents, pharmaceutical agents, nutraceutical agents, vitamins, minerals, medicaments and combinations thereof.

31. The composition of any one of the preceding claims, wherein said low viscosity vegetable gum has a viscosity of up to 10,000 cps.

32. The composition of any one of claims 1 to 30, wherein said low viscosity hydrolyzed vegetable gum has a viscosity of up to 5,000 cps.

33. The composition of any one of claims 1 to 30, wherein said low viscosity hydrolyzed vegetable gum has a viscosity of up to 500 cps.

34. The composition of any one of the preceding claims, wherein said low viscosity hydrolyzed vegetable gum comprises a galactomannan.
